# EUROPÄISCHE PATENTSCHRIFT

(11) **EP 3 598 989 B1**
(45) Veröffentlichungstag und Bekanntmachung des Hinweises auf die Patenterteilung: **06.08.2025**
(21) Anmeldenummer: 19190722.9
(22) Anmeldetag: 04.06.2009
(51) Int. Cl.: A61M 1/36

(54) **FALTBARER ORGANIZER FÜR BLUTSCHLAUCHELEMENTE**
COLLAPSIBLE ORGANIZER FOR BLOOD TUBE ELEMENTS
ORGANISATEUR PLIABLE POUR ÉLÉMENTS DE TUYAU SANGUIN

(30) Priorität: 05.06.2008 DE 102008026915
(43) Veröffentlichungstag der Anmeldung: 29.01.2020
(62) Teilanmeldung aus: 17182350.3
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Bobbert & Partner Patentanwälte PartmbB

(56) Entgegenhaltungen:
- WO-A1-00/47266
- GB-A- 2 271 963
- GB-A- 2 334 250
- US-A- 4 512 466
- US-A- 5 379 906
- US-A1- 2003 194 262
- US-A1- 2003 220 598
- US-A1- 2006 086 634
- US-B1- 6 536 156

## Beschreibung

Die vorliegende Erfindung betrifft einen Organizer gemäß dem Anspruch 1.

Aus der Praxis sind Vorrichtungen für eine extrakorporale Blutbehandlung bekannt. Zur Behandlung des Patienten wird ein Blutschlauchsystem mit entsprechenden Anschlüssen der Behandlungsvorrichtung verbunden. Das Blutschlauchsystem wird zu seinem besseren Handling an einem Organizer angeordnet, und zwar derart, dass die entsprechenden Elemente des Schlauchsystems für ein Anschließen des Schlauchsystems mit der Vorrichtung bereits in die richtige Position gebracht sind. Ein Organizer, wie er beispielsweise aus der WO 2004/032782 A1 bekannt ist, weist einen flexiblen, flächigen Grundkörper auf, an welchem Halte- bzw. Befestigungseinrichtungen zum Aufnehmen von einzelnen Bestandteilen oder Elementen des Schlauchsystems vorgesehen sind. Der Organizer gibt den flexiblen Schlauchsystemelementen somit sowohl Halt als auch eine günstige Position relativ zur Vorrichtung.

Zur Aufnahme von Elementen des Schlauchssystems muss der Organizer eine gewisse Größe aufweisen. Seine Abmessungen können dabei zur besseren Handhabung des Organizers beim Transport oder Lagern durch ein Aufrollen oder -falten verringert werden. Hierdurch wird jedoch auf Elemente des Schlauchsystems eine unerwünschte Belastung ausgeübt, und es kann zum Knicken oder Deformieren von Blutschlauchelementen kommen. Letzteres kann zu plastischen Verformungen führen und hierdurch Nachteile mit sich bringen.

Weiterhin lässt sich ein aufgerollter oder gefalteter Organizer des Standes der Technik schlecht handhaben, da er sich mangels klarer, fester Begrenzungsstrukturen sowie mangelnder Formstabilität nur schwierig mit der Hand greifen oder beim Transport oder beim Lagern Platz sparend stapeln lässt. Beim aufgerollten oder gefalteten Organizer kann ferner die Lage der einzelnen Schlauchelemente zueinander verloren gehen.

Aus der WO 00/47266 A1 ist ein in sich geschlossener Organizer für einen extrakorporalen Blutkreislauf bekannt.

Aufgabe der vorliegenden Erfindung ist es, einen weiteren Organizer anzugeben.

Die erfindungsgemäße Aufgabe wird gelöst durch die Merkmalskombination des Anspruchs 1.

So wird erfindungsgemäß ein Organizer vorgeschlagen, welcher wenigstens einen ersten, einen zweiten und einen dritten Abschnitt aufweist. Dabei ist der erste Abschnitt relativ zum zweiten Abschnitt bewegbar mit dem zweiten Abschnitt verbunden. Der zweite Abschnitt ist relativ zum dritten Abschnitt bewegbar mit dem dritten Abschnitt verbunden. Dabei kann der zweite Abschnitt - ebenso wie der erste und/ oder der dritte Abschnitt - mehrteilig ausgestaltet sein, wie dies beispielsweise in der angehängten Zeichnung zu erkennen ist. Der zweite Abschnitt kann dabei derart ausgestaltet sein, dass er einen definierten Abstand zwischen den mit ihm verbundenen Bereichen des ersten Abschnitts und des dritten Abschnitts zueinander sicherstellt. Abstandshalter zum Aufrechterhalten des Abstands zwischen dem ersten und dem dritten Abschnitt können vorteilhafterweise entbehrlich sein. Der zweite Abschnitt kann deren Funktion miterfüllen.

Unter einer relativen Bewegbarkeit wird erfindungsgemäß ein Schwenken, Falten, Biegen, Drehen oder dergleichen von zwei Abschnitten des Organizers zueinander verstanden. Dabei ist eine uneingeschränkte Bewegbarkeit jedoch nicht erforderlich. Die Bewegbarkeit zweier miteinander verbundener Abschnitte kann erfindungsgemäß durchaus aufgrund gegebener Materialsteifigkeit, durch zwischen den Abschnitten liegende Schlauchsystemelemente, und dergleichen beschränkt sein.

Unter einer Verbindung zwischen beispielsweise dem ersten Abschnitt und dem zweiten Abschnitt wird erfindungsgemäß jede Verbindung verstanden, mittels welcher ein Auseinanderfallen der jeweils miteinander verbundenen Abschnitte verhindert oder zu einem Verhindern beigetragen wird. Mittels der Verbindung kann eine Kraftübertragung zwischen den verbundenen Abschnitten möglich sein. Die Verbindung kann vorübergehender Art sein, sie kann jedoch auch die Lebensdauer des Organizers erreichen. Eine Verbindung im Sinne der vorliegenden Erfindung liegt auch dann vor, wenn miteinander verbundene Abschnitte mittels durchgehenden Materials, insbesondere einstückig, hergestellt sind. Die Verbindung kann flexibel ausgestaltet sein. Sie kann einen klar definierten, eckigen Übergang benachbarter Abschnitte zueinander erlauben oder sicherstellen. Die Verbindung kann einen variablen Abstand bspw. des ersten Abschnitts zum zweiten Abschnitt erlauben.

Weiterhin nimmt der Organizer aufgrund der äußeren Breite des zweiten Abschnitts im gefalteten Zustand eine Form an, an die eine Transportverpackung gut angepaßt werden kann. Dies wäre nicht der Fall, wenn der erste und der dritte Abschnitt, zwischen welchen sich die Komponenten des Blutschlauchsystems im zusammengeklappten Zustand des Organizers befinden, mit dem darin befindlichen Schlauchsystem direkt aufeinander liegen würden. Ein solch flexibles Paket hätte einen zu großen Bewegungsspielraum innerhalb einer, insbesondere starren, Verpackung. Das flexible Schlauchsystem allein besitzt kein reproduzierbares Dickenmaß und erschwert die Anpassung einer Verpackung.

Diese Eigenschaft der definierten Form des Organizers bzw. des definierten Abstands zumindest im Bereich des zweiten Abschnitts wird auch vorteilhaft genutzt beim Handling des Organizers durch den Benutzer. Dieser kann der Verpackung ein steifes, gefaltetes System entnehmen und dieses gut greifen, was erschwert wäre durch das Fehlen des klar definierten zweiten Abschnitts. Das dann "weich" verpackte Schlauchsystem ließe sich schlecht greifen.

Vorteilhafte Weiterbildungen des erfindungsgemäßen Gegenstands sind jeweils Gegenstand der Unteransprüche.

So wird in einer bevorzugten Ausführungsform ein Organizer vorgeschlagen, welcher zwischen dem ersten Abschnitt und dem zweiten Abschnitt einen Sollabwinkelbereich oder eine Vorzugs-Biegelinie oder -Klapplinie oder -Faltlinie aufweist. Ein solcher Sollabwinkelbereich oder dergleichen entspricht jenem Bereich, welcher ähnlich einem Gelenk die definierte Relativbewegung zwischen dem ersten Abschnitt und dem zweiten Abschnitt ermöglicht.

Indem sich der Sollabwinkelbereich aufgrund seiner besonderen Ausgestaltung für eine Gelenkwirkung zwischen dem ersten und dem zweiten Abschnitt anbietet, können der erste Abschnitt und insbesondere der zweite Abschnitt bei einem Zusammenklappen oder Zusammenfalten des Organizers ihre Form unverändert beibehalten. Eine Relativbewegung zwischen dem ersten Abschnitt und dem zweiten Abschnitt erlaubt somit vorteilhaft ein Zusammenklappen oder -falten des Organizers, ohne dass dazu der erste und/ oder der zweite Abschnitt ebenfalls gebogen, gefaltet oder geklappt werden müssten. Der Sollabwinkelbereich kann dabei einteilig oder mehrteilig ausgestaltet sein. Er kann ausgestaltet sein, um eine Abwinkelbarkeit bis zu einem festgelegten Winkel, bspw. 90°, zwischen den benachbarten Abschnitten zu erlauben.

Ein solcher Sollabwinkelbereich kann auch zwischen dem zweiten Abschnitt und dem dritten Abschnitt des erfindungsgemäßen Organizers dieser Ausführungsform vorgesehen sein.

In einer weiter bevorzugten Ausführungsform weist der erfindungsgemäße Organizer einen Sollabwinkelbereich mit einer Materialdicke oder -stärke auf, welche geringer ist als eine Materialdicke oder -stärke des ersten Abschnitts und/ oder des zweiten Abschnitts.

Bei dieser Ausführungsform ist das Bewegen des ersten Abschnitts relativ zum zweiten Abschnitt durch eine einfache und kostengünstig vorzusehende Verringerung der Materialdicke im Bereich des Sollabwinkelbereichs ermöglicht oder zumindest begünstigt.

In einer wiederum weiter bevorzugten Ausführungsform des erfindungsgemäßen Organizers ist der erste und/ oder der dritte Abschnitt durch wenigstens ein Filmscharnier mit dem zweiten Abschnitt verbunden. Das Filmscharnier kann hierbei eine oder mehrere, insbesondere zwei, Gelenkrillen aufweisen. Diese können voneinander beabstandet vorliegen. Das Filmscharnier erlaubt somit auf besonders zuverlässige Art ein definiertes Abknicken zweier mittels des Filmscharniers verbundener Abschnitte des Organizers.

Der zweite Abschnitt des Organizers kann in einer wiederum weiter bevorzugten Ausführungsform eine steifere Struktur als der erste Abschnitt oder der dritte Abschnitt aufweisen. Die steifere Struktur kann durch die Auswahl eines festeren Materials für den zweiten Abschnitt, durch ein dickeres Material im Bereich des zweiten Abschnitts in einer Richtung senkrecht zu einer Haupterstreckungsebene des zweiten Abschnitts oder durch das Vorsehen zusätzlicher, versteifend wirkender Elemente ausgestaltet sein.

Die steifere Struktur des zweiten Abschnitts dient vorteilhaft dazu, den durch den zweiten Abschnitt sichergestellten Abstand zwischen dem ersten Abschnitt und dem dritten Abschnitt - zumindest im Bereich ihrer Verbindung mit dem zweiten Abschnitt - im Gebrauch sicherzustellen. Der Grad der Steifigkeit des zweiten Abschnitts kann dabei derart durch konstruktive Maßnahmen bestimmt werden, dass der zweite Abschnitt seiner Abstandserhalterfunktion im normalen Gebrauch des Organizers gerecht wird, insbesondere ohne selber einzuknicken. Zudem ist durch die Steifigkeit des zweiten Abschnitts sichergestellt, dass in diesem Bereich verlaufende Blutschlauchelemente gegen ein Knicken gesichert sind.

In einer weiter bevorzugten erfindungsgemäßen Ausführungsform weist der zweite Abschnitt wenigstens ein Halteelement zum, insbesondere reversiblen, Aufnehmen von Blutschlauchteilen auf. Auch der erste und der dritte Abschnitt können Halteelemente dieser oder anderer Art aufweisen. Zu solchen Halteelementen zählen bspw. Schlauchhalter, Halter für venöse Klammern, etc.

Ein weiterer technischer Vorteil liegt erfindungsgemäß darin, dass Schlauchteile, die am ersten Abschnitt befestigt sind, dauerhaft knickfrei mit Schlauchteilen des dritten Abschnitts verbunden werden können, indem sie im zweiten Abschnitt durch ein zusätzliches Halteelement gestützt oder geführt sind. Wäre letzteres nicht der Fall, wie im Stand der Technik, so würden Verbindungen, welche sich über den ersten und den dritten Abschnitt erstrecken, zum Knicken neigen, mit den hiermit verbundenen Nachteilen.

In einer wiederum weiter bevorzugten Ausführungsform weist der erfindungsgemäße Organizer einen einstückig hergestellten Grundkörper auf, welcher den ersten Abschnitt, den zweiten Abschnitt und den dritten Abschnitt aufweist. Dies erlaubt eine besonders einfache und kostengünstige Herstellung des erfindungsgemäßen Organizers.

In einer wiederum weiter bevorzugten Ausführungsform weist der Organizer wenigstens ein Polystyrol auf oder ist hieraus gefertigt. Die Herstellung des Organizers kann mittels Thermoformen erfolgen.

In einer wiederum weiter bevorzugten Ausführungsform weist der Sollabwinkelbereich eine Materialdicke auf, welche geringer ist als eine Materialdicke des ersten Abschnitts und/ oder des zweiten Abschnitts und/ oder des dritten Abschnitts.

In einer wiederum weiter bevorzugten Ausführungsform ist der zweite Abschnitt mehrteilig ausgestaltet.

In einer wiederum weiter bevorzugten Ausführungsform weist der Organizer einen einstückig hergestellten Grundkörper auf, welcher den ersten Abschnitt, den zweiten Abschnitt und den dritten Abschnitt aufweist.

Die vorliegende Erfindung wird im Folgenden exemplarisch erläutert mit Bezug auf die beigefügte Figur. In der Figur gilt:
Fig. 1 zeigt einen faltbaren Organizer gemäß der vorliegenden Erfindung in einem gefalteten Zustand; und
Fig. 2 zeigt den Organizer aus Fig. 1 im geöffneten Zustand.

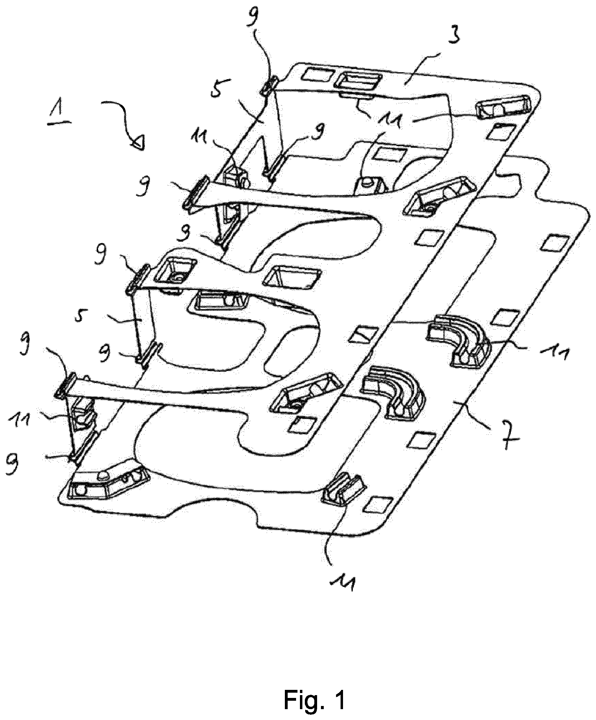

Fig. 1 zeigt einen faltbaren Organizer 1 mit einem ersten Abschnitt 3, einem zweiten Abschnitt 5 sowie einem dritten Abschnitt 7. Der erste Abschnitt 3 und der dritte Abschnitt 7 sind dabei mittels der Stützwirkung des zweiten Abschnitts 5 an ihren jeweils zum linken Bildrand hin gelegenen Enden voneinander beabstandet und zugleich miteinander verbunden. Die Verbindung zwischen erstem Abschnitt 3 und zweitem Abschnitt 5 erfolgt mittels mehrerer Sollabwinkelbereiche 9. Der zweite Abschnitt 5 ist mit dem dritten Abschnitt 7 ebenfalls mittels Sollabwinkelbereiche 9 verbunden.

Fig. 1 ist zu entnehmen, dass jeder der drei Abschnitte 3, 5 und 7 jeweils mehrere Halteelemente 11 zum Befestigen von in Fig. 1 nicht gezeigten Blutschlauchsystemelementen aufweist. Die Halteelemente 11 des zweiten Abschnitts 5 bewirken aufgrund ihrer Verbindung mit dem zweiten Abschnitt 5 bereits eine Versteifung des zweiten Abschnitts 5. Ferner dienen auch in die Halteelemente 11 des zweiten Abschnitts 5 aufgenommene - in Fig. 1 nicht gezeigte - Blutschlauchsystemelemente einer weiteren Versteifung des zweiten Abschnitts 5.

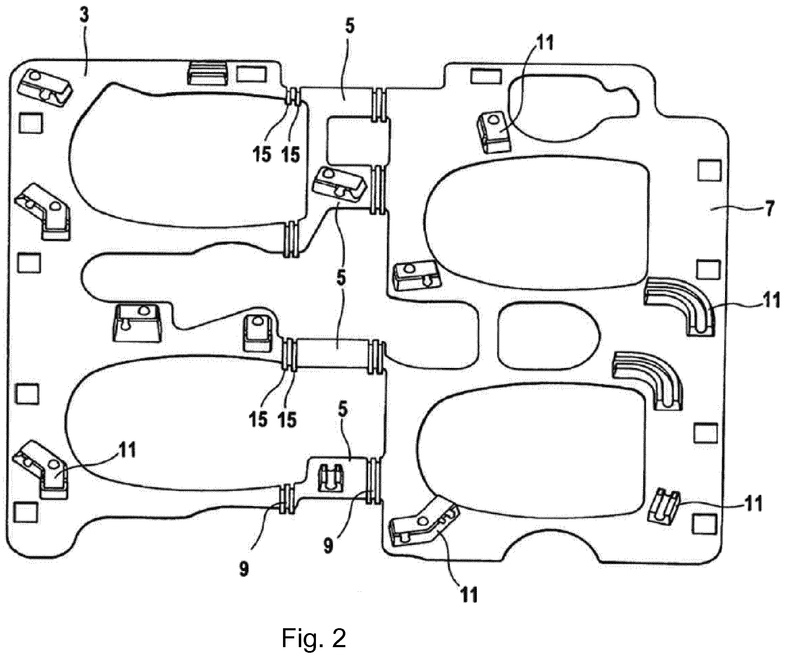

Fig. 2 zeigt den Organizer 1 aus Fig. 1 in einem ausgebreiteten oder aufgeklappten Zustand. Zu erkennen ist, dass der erste Abschnitt 3, der zweite Abschnitt 5 und der dritte Abschnitt 7 einstückig aus einem Grundkörper 13 hergestellt sind.

Die Sollabwinkelbereiche 9 weisen jeweils ein Filmscharnier mit exemplarisch je zwei Gelenkrillen 15 auf. Sie können dergestalt sein, dass sie ein Abknicken der Abschnitte zueinander nicht über einen vorbestimmten Winkel hinaus erlauben, was vorteilhaft zu erhöhter Festigkeit des Organizers beitragen kann.

## Patentansprüche

1. Organizer (1) mit wenigstens einem ersten, einem zweiten und einem dritten Abschnitt, wobei der erste Abschnitt (3) relativ zum zweiten Abschnitt (5) bewegbar mit dem zweiten Abschnitt (5) verbunden ist, wobei der zweite Abschnitt (5) relativ zum dritten Abschnitt (7) bewegbar mit dem dritten Abschnitt (7) verbunden ist, und wobei der Organizer (1) derart ausgestaltet ist, dass Schlauchteile, wenn sie am ersten Abschnitt (3) befestigt werden, dauerhaft knickfrei mit Schlauchteilen des dritten Abschnitts (7) verbindbar sind, indem sie im zweiten Abschnitt (5) durch ein zusätzliches Halteelement (11) gestützt oder geführt werden.

2. Organizer (1) nach Anspruch 1, wobei zwischen dem ersten Abschnitt (3) und dem zweiten Abschnitt (5) und/ oder zwischen dem zweiten Abschnitt (5) und dem dritten Abschnitt (7) wenigstens ein Sollabwinkelbereich (9) ausgestaltet ist.

3. Organizer (1) nach Anspruch 2, wobei der Sollabwinkelbereich (9) eine Gelenkwirkung zwischen dem ersten (3) und dem zweiten Abschnitt (5) ermöglicht.

4. Organizer (1) nach Anspruch 3, mit einem weiteren Sollabwinkelbereich (9), welcher eine Gelenkwirkung zwischen dem zweiten (5) und dem dritten Abschnitt (7) ermöglicht.

5. Organizer (1) nach Anspruch 2, 3 oder 4, wobei der Sollabwinkelbereich (9) und/oder der weitere Sollabwinkelbereich (9) mehrteilig ausgestaltet sind.

6. Organizer (1) nach einem der Ansprüche 2 bis 5, welche eine Abwinkelbarkeit bis zu einem festgelegten Winkel zwischen den benachbarten Abschnitten (3, 5, 7) erlaubt.

7. Organizer (1) nach einem der vorangegangenen Ansprüche, wobei der dritte Abschnitt (7) mit dem ersten Abschnitt (3) nur über den zweiten Abschnitt (5) verbunden ist.

8. Organizer (1) nach einem der vorangegangenen Ansprüche, wobei der zweite Abschnitt (5) eine steifere Struktur als der erste Abschnitt (3) und/ oder der dritte Abschnitt (7) aufweist.

9. Organizer (1) nach Anspruch 8, wobei die steifere Struktur durch das Vorsehen zusätzlicher, versteifend wirkender Elemente ausgestaltet ist.

10. Organizer (1) nach einem der vorangegangenen Ansprüche, wobei der erste Abschnitt (3) und der dritte Abschnitt (7) wenigstens je ein Halteelement (11) zum reversiblen Aufnehmen von Blutschlauchsystemelementen aufweist.

11. Organizer (1) nach einem der vorangegangenen Ansprüche, wobei der zweite Abschnitt (5) derart ausgestaltet ist, dass er einen definierten Abstand zwischen den mit ihm verbundenen Bereichen des ersten Abschnitts (3) und des dritten Abschnitts (7) zueinander sicherstellt.

12. Organizer (1) nach einem der vorangegangenen Ansprüche, wobei der zweite Abschnitt (5) wenigstens ein Halteelement (11) zum reversiblen Aufnehmen von Blutschlauchsystemelementen aufweist.

13. Organizer (1) nach einem der vorangegangenen Ansprüche, wobei Schlauchteile, die am ersten Abschnitt (3) befestigt sind, mittels eines zusätzlichen Halteelements (11) des zweiten Abschnitts (5) mit Schlauchteilen des dritten Abschnitts (7) verbunden sind.

14. Organizer (1) nach einem der vorangegangenen Ansprüche, wobei das Halteelement (11) als Schlauchhalter ausgestaltet ist.

15. Organizer (1) nach einem der vorangegangenen Ansprüche, welcher mit Blutschlauchsystemelementen bestückt ist.

16. Organizer (1) nach einem der vorangegangenen Ansprüche, bei welchem die Blutschlauchsystemelemente im zusammengeklappte Zustand des Organizers (1) zwischen dem ersten Abschnitt (3) und dem dritten Abschnitt (7) angeordnet sind.

## Claims

1. An organizer (1) comprising at least a first, a second and a third section, wherein the first section (3) is connected to the second section (5), movably relative to the second section (5), wherein the second section (5) is connected to the third section (7), movably relative to the third section (7), and wherein the organizer (1) is designed such that tube parts, when attached to the first section (3), can be permanently connected without kinking to tube parts of the third section (7), by being supported or guided in the second section (5) by an additional holding element (11).

2. The organizer (1) according to claim 1, wherein at least one target angle area (9) is designed between the first section (3) and the second section (5) and/or between the second section (5) and the third section (7).

3. The organizer (1) according to claim 2, wherein the target angle area (9) enables a hinge action between the first (3) and the second section (5).

4. The organizer (1) according to claim 3, with a further target angle area (9), which enables a hinge action between the second (5) and the third section (7).

5. The organizer (1) according to claim 2, 3 or 4, wherein the target angle area (9) and/or the further target angle area (9) are designed in several parts.

6. The organizer (1) according to any one of claim 2 to 5, which allows an angle deviation up to a defined angle between the adjacent sections (3, 5, 7).

7. The organizer (1) according to any one of the preceding claims, wherein the third section (7) is connected to the first section (3) only via the second section (5).

8. The organizer (1) according to any one of the preceding claims, wherein the second section (5) comprises a stiffer structure than the first section (3) and/or the third section (7).

9. The organizer (1) according to claim 8, wherein the stiffer structure is designed by providing additional stiffening elements.

10. The organizer (1) according to any one of the preceding claims, wherein the first section (3) and the third section (7) each comprise at least one holding element (11) for reversibly receiving blood tubing system elements.

11. The organizer (1) according to any one of the preceding claims, wherein the second section (5) is designed such that it ensures a defined distance to each other between the areas of the first section (3) and those of the third section (7) that are connected to it.

12. The organizer (1) according to any one of the preceding claims, wherein the second section (5) comprises at least one holding element (11) for reversibly receiving blood tubing system elements.

13. The organizer (1) according to any one of the preceding claims, wherein tube parts which are attached to the first section (3) are connected to tube parts of the third section (7) by means of an additional holding element (11) of the second section (5).

14. The organizer (1) according to any one of the preceding claims, wherein the holding element (11) is designed as a tube holder.

15. The organizer (1) according to any one of the preceding claims, which is equipped with blood tubing system elements.

16. The organizer (1) according to any one of the preceding claims, in which the blood tubing system elements are arranged between the first section (3) and the third section (7) in the collapsed state of the organizer (1).

## Revendications

1. Un organisateur (1) comprenant au moins une première,
une seconde ainsi qu'une troisième section,
où la première section (3) est reliée à la seconde section (5) de manière mobile par rapport à la seconde section (5), où la seconde section (5) est reliée à la troisième section (7) de manière mobile par rapport à la troisième section (7), et où l'organisateur (1) est conçu de telle sorte que des pièces de tuyau, lorsqu'elles sont attachées à la première section (3), peuvent être reliées, de manière permanente sans plis, à des pièces de tuyau de la troisième section (7), en étant soutenues ou guidées dans la seconde section (5) par un élément de retenue supplémentaire (11).

2. L'organisateur (1) selon la première revendication, où au moins une zone angulaire cible (9) est conçue entre la première section (3) et la seconde section (5) et/ou entre la seconde section (5) et la troisième section (7).

3. L'organisateur (1) selon la revendication 2, où la zone angulaire cible (9) permet une action de charnière entre la première (3) et la seconde section (5).

4. L'organisateur (1) selon la revendication 3, comprenant une zone angulaire cible (9) supplémentaire, qui permet une action de charnière entre la seconde (5) et la troisième section (7).

5. L'organisateur (1) selon la revendication 2, 3 ou 4, où la zone angulaire cible (9) et/ou la zone angulaire cible (9) supplémentaire sont conçues en plusieurs parties.

6. L'organisateur (1) selon l'une quelconque des revendications 2 à 5, qui permet une déviation angulaire jusqu'à un angle défini entre les sections adjacentes (3, 5, 7).

7. L'organisateur (1) selon l'une quelconque des revendications précédentes, où la troisième section (7) est reliée à la première section (3) uniquement via la seconde section (5).

8. L'organisateur (1) selon l'une quelconque des revendications précédentes, où la seconde section (5) présente une structure plus rigide que la première section (3) et/ou la troisième section (7).

9. L'organisateur (1) selon la revendication 8, où la structure plus rigide est conçue par la fourniture d'éléments de renforcement supplémentaires.

10. L'organisateur (1) selon l'une quelconque des revendications précédentes, où la première section (3) et la troisième section (7) comprennent chacune au moins un élément de retenue (11) pour recevoir de manière réversible des éléments du système de tuyaux sanguins.

11. L'organisateur (1) selon l'une quelconque des revendications précédentes, où la seconde section (5) est conçue de telle sorte qu'elle assure une distance définie entre les zones de la première section (3) et celles de la troisième section (7) qui lui sont reliées.

12. L'organisateur (1) selon l'une quelconque des revendications précédentes, où la seconde section (5) comprend au moins un élément de retenue (11) pour recevoir de manière réversible des éléments du système de tuyaux sanguins.

13. L'organisateur (1) selon l'une quelconque des revendications précédentes, où les pièces de tuyau attachées à la première section (3) sont reliées aux pièces de tuyau de la troisième section (7) au moyen d'un élément de retenue supplémentaire (11) de la seconde section (5).

14. L'organisateur (1) selon l'une quelconque des revendications précédentes, où l'élément de retenue (11) est conçu comme un support de tuyau.

15. L'organisateur (1) selon l'une quelconque des revendications précédentes, qui est équipé d'éléments du système de tuyaux sanguins.

16. L'organisateur (1) selon l'une quelconque des revendications précédentes, où les éléments du système de tuyaux sanguins sont agencés entre la première section (3) et la troisième section (7) dans l'état replié de l'organisateur (1).
